# EUROPEAN PATENT SPECIFICATION

(11) **EP 3 685 811 B1**
(45) Date of publication and mention of the grant of the patent: **27.03.2024**
(21) Application number: 17926275.3
(22) Date of filing: 22.09.2017
(51) Int. Cl.: A61F 13/42, A61F 13/58

(54) **URINE-DETECTION DEVICE WITH ADHESIVE TAPE AND EMITTER SENSOR**
URINDETEKTIONSVORRICHTUNG MIT KLEBEBAND UND EMITTERSENSOR
DISPOSITIF POUR LA DÉTECTION D'URINE AVEC RUBAN ADHÉSIF ET CAPTEUR ÉMETTEUR

(43) Date of publication of application: 29.07.2020
(73) Proprietor: Muñoz Herencia, Alberto, 03440 Ibi Alicante (ES)
(72) Inventor: Muñoz Herencia, Alberto, 03440 Ibi Alicante (ES)
(74) Representative: Ibarra Garcia, Isabel
(86) International application number: PCT/ES2017/000118
(87) International publication number: WO 2019/058002

(56) References cited:
- EP-A1- 3 143 975
- GB-A- 2 512 575
- US-A- 3 143 208
- US-A- 4 106 001
- US-A- 4 704 108
- US-A- 4 768 023
- US-A- 5 469 145
- US-A- 5 469 145
- US-A- 5 904 671
- US-A1- 2008 269 702

## Description

### TECHNICAL FIELD

The invention herein proposed belongs to the sector relating to everyday necessities of life, in the chapter on health and protection in reference to medical sciences and hygiene, involved directly in the clinical and pharmaceutical industry concerning the manufacture of accessories for ancillary garments for sick people, the elderly, and children.

### BACKGROUND OF THE INVENTION

Urinary incontinence is a health-related aspect that usually affects small children, children suffering enuresis, the elderly, or dependants or people hospitalised in clinics with mobility issues due to a number of causes.

To prevent its harmful effects on the body, clothes, or, where appropriate, bedding, the most common solution is the use of single-use nappies, sanitary towels, or absorbent garments which are disposed of once they get wet. Although nappies with a high absorption capacity to reduce as much as possible the action of urine on skin, particularly in the case of very small children and the elderly are currently being made, it is always advisable to detect as quickly as possible when a nappy is wet in order to replace it with a clean, dry one for basic hygienic reasons.

It is understood that when the person wearing a nappy does not have the ability to inform someone that it needs to be changed, there is a need to come up with suitable means generating the proper warning so that this person's caregiver can provide the attention required in such situation.

In that sense, several solutions registered in patent offices that solve the problem being contemplated are known. Several very similar solutions are mentioned below:
- US 20040004548 A1. Electronic diaper
- US 20100277324 A1. Diaper with urine sensor
- US 5469145 A. Wet diaper detector

The first example describes an electronic diaper with a sensor and controller detecting the dampness in the diaper and giving a warning by means of an LED lamp or an acoustic alarm. It is complemented with a timer in order to know how much time has gone by since the wetting of the diaper until the caregiver changes it.

The second example describes a type of diaper with a sensing line incorporated in the absorption layer which ends in a bag in which a detector is introduced. By changing the impedance of the line, a light, acoustic, or vibratory warning signal is generated. The sensor is reusable.

Lastly, the third example shows a solution consisting of a strip of two conductors incorporated in the diaper. The sensor is connected at the ends of the strip, detecting changes in the resistor or in the capacitive value between conductors, activating the warning signal. When the diaper is changed, the sensor is retrieved and the diaper is disposed of with the strip.

The three solutions are very similar, as they correspond to the specific case of urine detection by means of special diapers incorporating sensing lines.

Other devices are known in the art. For instance, US5469145A discloses a urine-detection device with adhesive tape and an emitter sensor, the device does not include a clamp-type supporting member.

US5904671A discloses a tampon wetness detection system with control buttons and GB2512575A discloses a system for detecting and monitoring incontinence which comprises means for detecting a gas associated with incontinence, means for transmitting the detected gas level to a central controller; means for comparison of the detected gas level with a predetermined reference level; means for transmitting a signal to a receiving device if the detected gas level is equal to or exceeds the reference level. None of these documents anticipates a device formed by the components of the present invention.

US3143208A discloses an adhesive tape and EP3143975A1 discloses a smart nursing consumable, which includes an absorbent body and a sensor module disposed on the absorbent body. The sensor module includes a first film sheet, at least one wire, a second film sheet and a conductive pad. The first film sheet is disposed on the absorbent body. However, the device does not include a clamp-type supporting member.

In view of these background documents, the inventor has devised the device described here, having a simple composition, very positive practical results, and an affordable price for most economies, making it an interesting novel invention with a high potential for its future distribution and marketing. The invention is particularly useful for being applied in hospitals and health centres where a considerable number of patients are cared for, and it is possible to centralise the information and swiftly and effectively take care of the alarms that are given off.

### DISCLOSURE OF THE INVENTION

Taking into account the background documents disclosed above and others referring to more complex devices existing on the market, the inventor has devised the idea of applying a special adhesive tape on any type of nappy, sanitary towel, or pad of the type normally used medical centres, hospitals, nursing homes, retirement homes, or private residences.

Nappies and pads detecting urine sold on the market are higher-priced items than normal nappies and pads as they must incorporate the corresponding installation during the manufacturing process. As there are multiple qualities, types, and sizes, it is understood that their price is considerably higher with respect to that of ordinary nappies or pads.

The invention is defined in claim 1. Preferred features are enclosed in the dependent claims.

In a first, very simple solution, the invention shows a tape having a width in the order of 1.5-2.5 centimetres, incorporating two considerably parallel electrical conductors, one end of which is arranged to establish proper electrical contact on the connection terminals of an emitter sensor duly housed and protected by a clamp-type device.

With this arrangement, the emitter sensor is prepared for taking readings of ohmic resistance in the range between 0.1 and 100 KΩ, which means that with the supporting member/emitter sensor unit correctly placed, the following may occur:
- High reading → very weak current between the parallel conductors → dry nappy → no attention required
- Low reading → sensitive current between conductors → wet nappy →requires being replaced with a dry one

Under these conditions, if the user removes the supporting member/emitter sensor by pulling on it, or if the caregiver has not properly put said unit in place and this has not been checked, the reading of the resistor that is shown will be high (highest point on the scale due to an open circuit) and, however, the user requires immediate attention to normalize the placement of the supporting member/emitter sensor. In other words, this simplest solution, without denying the advantages it has, has an important drawback that the inventor has corrected and improved by incorporating a resistor between both parallel conductors at the end nearest the location where the supporting member/emitter sensor is clamped.

With this improved embodiment, the circuit for reading the ohmic resistance of the sensor is calibrated such that it is capable of distinguishing three very specific different cases:
- Supporting member/emitter sensor unit properly placed → reading between 95 KΩ and 30 KΩ → dry nappy → no attention required
- Supporting member/emitter sensor unit properly placed → reading ≤ 30 KΩ → sensitive current between conductors → wet nappy → nappy requires being replaced. The device recognises three degrees of dampness: high, medium, and low. The caregiver can therefore weigh the urgency of intervention
- Supporting member/emitter sensor unit improperly placed or removed by the user → reading > 95 KΩ (highest point on the scale) (open circuit) → immediate attention required

The tape is made from a normal textile material or, alternatively, from a material of propylene of the type known as "non-woven fabric," which is more economical and can be of the ecological type, with the subsequent advantages for purposes of environmental pollution.

In any of the cases, the tape incorporates an adhesive on one of its faces with a protective band that is removed when it is adhered on the nappy.

The inventor envisages the marketing of the tape in two forms:
In the first form, the tape is provided in groups of strips having specific lengths in the order of 50 centimetres, with the resistor incorporated at one end.

In a second form, the tape is provided wound, as if it were a roll of surgical tape, in lengths of several metres to be cut into equal segments of 50 centimetres, where the cut must always be made around the notch where the resistor is located.

The mentioned length to be precisely defined in due time must be slightly greater than the largest size of the nappies normally used, which may entail the need to cut certain excess at the end opposite where the resistor is located.

Its use is very simple, as it consists of cutting the necessary length and adhering on any type of nappy, or even on commonly used garments such as panties or underwear.

The end of the resistor is arranged in the front part of the nappy so that it is close to the notch where the emitter sensor is connected with its clamp device.

In the embodiment shown, the emitter sensor, with its corresponding electronic circuit, has an activation button and three LEDs powered by an internal battery. When the LEDs are off, the nappy is completely dry. If one of them lights up, it shows the existence of slight dampness. If two of them light up, it shows greater dampness, and if the three of them light up, the nappy must be replaced immediately.

These indications are useful when the caregiver directly checks the patient's conditions, but in cases of a significant number of patients, supervision is performed remotely as each of the corresponding emitter sensors sends all the signals to the control centre where visually monitoring is performed on a screen or with acoustic warnings.

In summary, the device of the invention has, among others, the following advantages:
- It reduces complications resulting from the use of sanitary towels for incontinence, such as urinary infections or ulcers.
- It reduces hospital stays, painful medical care, and treatments with antibiotics that weaken the patient.
- Since frequent preventive checks are not needed, it prevents conflicts with patients suffering from dementia and helps to preserve their dignity.
- It reduces the physical and psychological stress of the caregiver, who may dedicate more time and mental space to offer company to the patient.
- Overnight or during sleep periods, the patient does not have to be woken up to perform checks.
- It allows the simultaneous monitoring of several patients through a control centre.
- The statistical data that is gathered provided an overview of incontinence management and allows optimising the organisation of the work equipment, with the subsequent savings in time resources.

The following section, which must be considered an integral part of this document, includes several schematic drawings that describe the invention.

### BRIEF DESCRIPTION OF THE DRAWINGS

To complement the preferred embodiment described below and for the purpose of helping to better understand the features of the invention, a set of drawings, which must always be interpreted in an illustrative and non-limiting manner, is attached as an integral part of said description.
Figure 1 schematically shows the tape of the invention presented in the form of a strip, where the following is indicated:
   1. Tape strip
   2. Conductor
   3. Adhesive layer
   4. Protective sheet
Figure 2 schematically shows a nappy with a tape strip that is adhered along its middle area, where it can be seen that the resistor is close to the front end. An enlarged view is shown for better viewing purposes.
   5. Nappy
   11. Resistor
   12. Contact point
   13. Pre-marked fold
Figure 3 shows the nappy unit and an emitter sensor with its supporting member before it is coupled on the nappy.
   6. Emitter sensor
   7. Control button
   9. Indicator LED
   10. Supporting member
Figures 4, 5 and 6 schematically show the nappy, the tape strip with its resistor, and the emitter sensor in situations reflecting a dry nappy (Figure 4), a wet nappy (Figure 5), and an improperly placed sensor (Figure 6), where the following is indicated:
   8. Connection terminal
   5.1. Wet nappy
   14. Ohmic reader
Figure 7 shows the tape of the invention presented in the form of a roll
   1.1. Tape roll
   13.1. Pre-marked cut
Figure 8 separately depicts a front view of the emitter sensor and the supporting member which will serve for obtaining a firm attachment on the nappy.
Figure 9 shows a rear view of the same elements as in the preceding figure.
   8. Connection terminal

### PREFERRED EMBODIMENT OF THE INVENTION

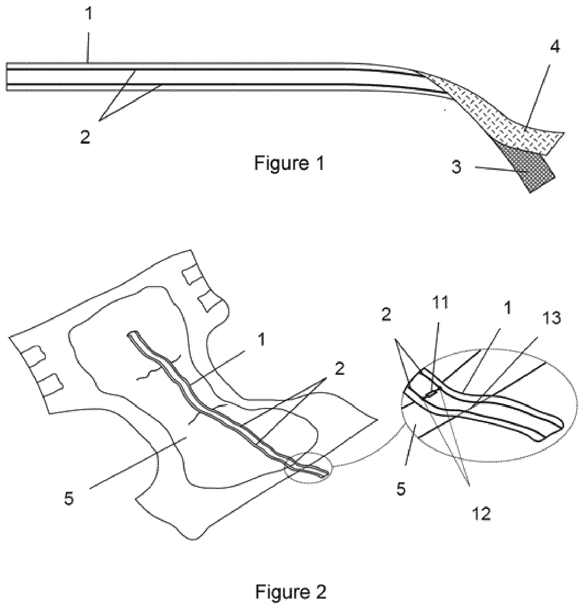

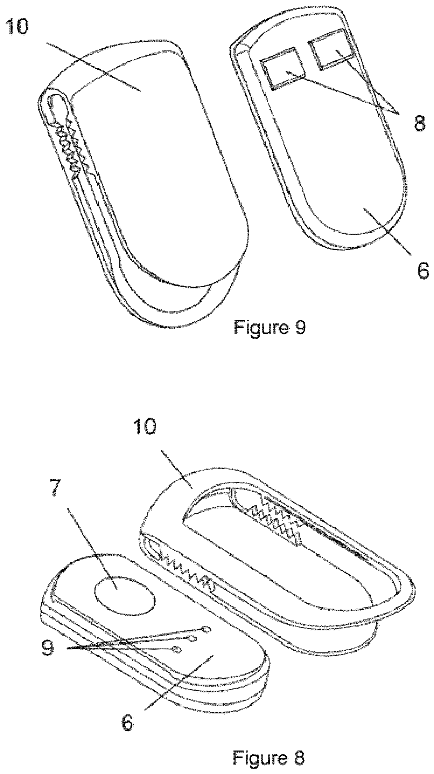

The urine-detection device with adhesive tape and an emitter sensor (Figures 1 to 9) allows the easy provision of a unit urine detector, preventing the need for higher-priced special nappies, sanitary towels, or pads.

In an embodiment preferred by the inventor, said invention comprises two well distinguished parts that complement one another and are referred to as tape and an emitter sensor. As will be shown, the tape is presented in the two forms of tape strip (1) and tape roll (1.1)

The tape strip (1) (Figure 1) is shown for supplying packs or groups of independent strips having a length of about 50 centimetres, which is slightly larger than the largest-sized nappy available on the market in this field. It has an approximate width of 1.5-2.5 centimetres, being equipped on one face with two longitudinal conductors (2) made of copper, aluminium, steel, or a similar material which, developed in parallel, are situated a few millimetres from the edges of the tape and are integrally jointed to same by any common method, such as adhering, stitching, stamping, or the like. The other face of the tape strip (1) is equipped with an adhesive layer (3), which can be continuous or discrete (with notches or lines), such as in surgical tape, to favour the permeability, said strip being protected by means of a protective sheet (4) of paper or thin plastic until the latter is removed when the strip is applied on the nappy (5).

A 90 KΩ resistor (11) with contact points (12) on each of the conductors (2) is connected close to one end of the tape strip (1), as can be observed (Figure 2), where the pre-marked fold (13) is also shown.

According to these features, the invention is simple to use and consists of taking a tape strip (1), removing the protective sheet (4), and adhering it on a nappy (5), taking care that the resistor (11) is located in the front part, matching up the pre-marked fold (13) along the edge of the nappy and folding the tape strip (1) over so as to slightly catch the nappy (5). Lastly, the excess tape is cut at the opposite end so that it can cover the entire length. It is understood that there will be more or less excess, depending on the size of the nappy.

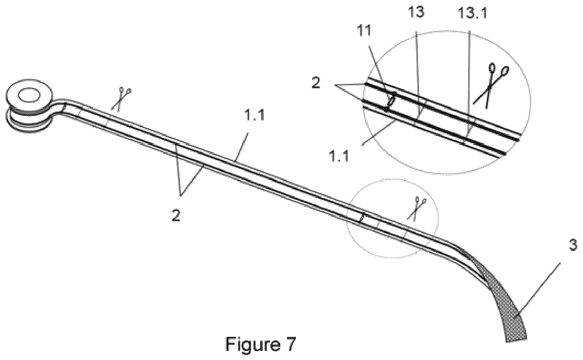

The form of the tape roll (1.1) (Figure 7) consists of a continuous tape identical to the tape strip (1) which is wound as if it were a roll of surgical tape, with the particularity that is has resistors (11) connected on the conductors (2) separated by about 50 centimetres, which coincides with the length of the tape strip (1). In this case, in addition to the pre-marked folds (13) there are also pre-marked cuts (13.1), such that the use thereof can be identical to the use of the tape strip (1) by simply cutting along the mentioned pre-marked cuts (13.1). In this case, the protective sheet (4) is dispensed with, offering the subsequent savings, as the tape protects itself.

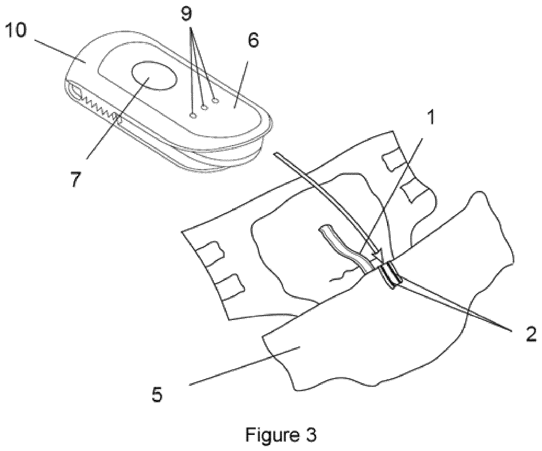

The emitter sensor (6) (Figures 3, 8 and 9) is an electronic device which, embedded in the support (10), is designed so that the connection terminals (8) thereof establish proper electrical contact with the conductor (2), therefore the separation between the centres of said connection terminals (8) is identical to the separation between the conductors (2).

The way to couple the emitter sensor (6) to the nappy (5) once the corresponding tape strip (1) has been adhered on same and folding along the pre-marked fold (13) has been performed is schematically shown (Figure 3). Immobility of the attachment is assured by the clamp effect of the supporting member (10) where the emitter sensor (6) is housed.

The emitter sensor (6) is equipped with electronic circuits that read the conductivity variation between conductors (2), the generation of alarm signals, and the sending, where appropriate, of such signals to a remote concentrator.

It is externally equipped with the activation button (7), with one, two, or more indicator LEDs (9) and the connection terminals (8) mentioned above.

As a whole, the device of the invention is devised to continuously measure, once installed on the patient, conductivity between the conductors (2) by the reading of the ohmic resistance between same.

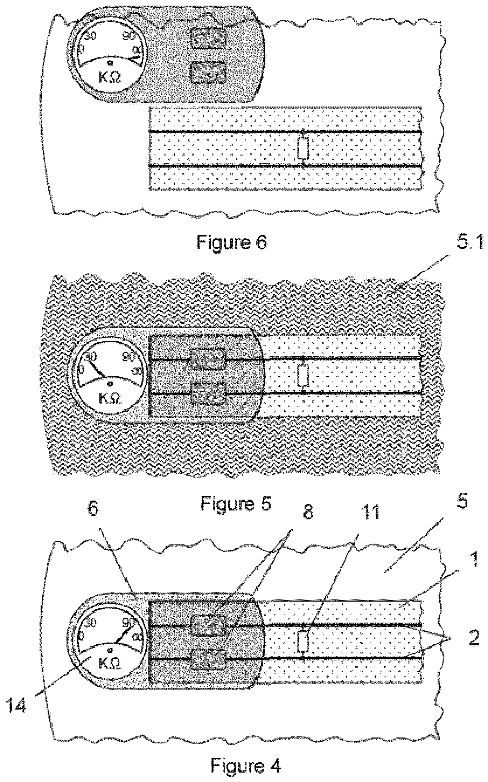

To better understand the invention, the three depicted cases are schematically shown in detail (Figures 4, 5 and 6).

Once case (Figure 4) corresponds to the initial situation of an emitter sensor (6) correctly placed on the tape strip (1) with its connection terminals (8) properly centred on the conductors (2) with the nappy (5) recently put on the user being dry. Under those conditions, the ohmic reader (14) of the emitter sensor (6), which is shown in the form of a display with a scale for greater clarity, offers a reading of 90 KΩ, which corresponds to the value of the resistor (11) assembled in parallel on the conductor (2). It is therefore a case of normality which, in practice, is checked by means of pushing the control button (7) (Figure 3) with the LEDs (9) flashing. The device remains in standby until the situation shown (Figure 5) reflecting a wet nappy (5.1), where the ohmic reader (14) offers a reading of about 30 KΩ or less, depending on the amount of urine released by the user. When those circumstances occur, in addition to the resistor (11), another virtual resistor between the conductors (2) appears due to the presence of urine which is theoretically assembled in parallel with the resistor (11), giving a total resistance result < 30 KΩ, generating the warning that it is necessary to change the nappy.

The depicted case (Figure 6) is also possible, where, due to a poor initial insertion, due to accidental or even bad faith movements or pulling by the user, the emitter sensor (6) comes out of place, without there being contact between the connection terminals (8) and the conductors (2). In that case, the ohmic reader (14) gives a reading in KΩ reaching the highest point on the scale, which is equivalent to the open circuit situation, regardless of whether the nappy (5) is dry or wet (5.1), where it is evident that the user requires attention.

Proper placement can be checked "in-situ" by the caregiver by pushing, as indicated above, the control button (7) with the corresponding activation of one of the LEDs (9), which flashes. If the emitter sensor is improperly placed, the lateral LEDs (9) will blink three times after pushing the control button (7).

In all cases, both the situation of normality and any other anomalous situation can be detected remotely in a control centre given that the emitter sensor (6) is wirelessly connected to a panel of said centre where acoustic and/or light signals are received, continuously recording all episodes.

The device of the invention is applicable to any number of users, which is very useful in the case of hospitals, nursing homes for the elderly, or health centres, making it possible to manage the signals over Internet or intranet networks and being concentrated in a central monitoring post.

The possibility of applying the tape strip (1) or tape roll (1.1) on any type of nappy (5), sanitary towel, or pad, whatever the type and size may be, resulting in obvious overall savings, is also very advantageous. As devised by the inventor, the tape in question can be applied to any other type of undergarment.

It is not considered necessary to further extend the content of this description for one skilled in the art to understand its scope and the advantages derived from the invention, and to develop and carry out to practice the object thereof. However, it must be understood that the invention has been described according to a preferred embodiment thereof, so it is susceptible to modifications without this entailing any alteration whatsoever of the basis of said invention, where such modifications may affect the shape, size, and/or manufacturing materials; that is, the terms in which this preferred description of the invention has been disclosed must always be interpreted in a broad and non-limiting manner.

## Claims

1. A urine-detection device with adhesive tape and an emitter sensor, the device being **characterised in that** it comprises a tape strip (1) equipped with two parallel, longitudinal metallic conductors (2) to which a 90 KΩ resistor (11) is connected by means of superposition, adhesion or printing, the resistor being situated at one end of the tape strip (1), close to a pre-marked fold (13), near an area intended to connect a unit formed by the support (10) and the emitter sensor (6), which produces in-situ visual warnings by means of LEDs (9) when a control button is pressed (7) and which is programmed to continually transmit data to a control centre panel by means of a wireless connection, to facilitate normality information and information regarding the need for attention because of a wet nappy (5.1), where the emitter sensor (6) internally houses the electronic circuit for reading the resistors between the conductor (2), the alarm generator/emitter and the power supply battery externally presenting the control button (7), one, two or more indicator LEDs (9), and two connection terminals (8) operating while inserted in a clamp-type supporting member (10) for immobilising same on nappies, sanitary towels, pads, or any other garment.

2. The urine-detection device with adhesive tape and an emitter sensor according to claim 1, **characterised in that** the tape strip (1) is presented as a wound tape roll (1.1), with a plurality of resistors (11) which is connected to the conductors (2), separated by a distance of about 50 centimetres with pre-marked folds (13) and pre-marked cuts (13.1).

3. The urine-detection device with adhesive tape and an emitter sensor according to claims 1 and 2, **characterised in that** the tape strips (1) or tape roll (1.1) are made of textile material, plastic material, non-woven fabric or any other material, and the two conductors (2), which are integrally joined together by one of their faces by any method, are made of copper, aluminium, steel, or a similar metal. The opposite face of the tapes has an adhesive layer (3), said layer being continuous or discrete due to the presence of notches or lines to increase its permeability.

4. The urine-detection device with adhesive tape and an emitter sensor according to the preceding claims, **characterised in that** the conductors (2) of the tape strip (1) or tape roll (1.1) establish contact with the emitter sensor (6) through the connection terminals (8).

5. The urine-detection device with adhesive tape and an emitter sensor according to the preceding claims, **characterised in that** the tape strip (1) is presented in segments having lengths of about 50 centimetres, the adhesive layer (3) being protected with a protective sheet of paper or plastic.

## Patentansprüche

1. Urindetektionsvorrichtung mit Klebeband und einem Emittersensor, wobei die Vorrichtung **dadurch gekennzeichnet ist, dass** sie einen Bandstreifen (1) umfasst, welcher mit zwei parallelen, länglichen metallischen Leitern (2) ausgerüstet ist, an welche ein 90 KΩ-Widerstand (11) mittels Überlagerung, Anhaftung oder Drucken angeschlossen ist, wobei sich der Widerstand an einem Ende des Bandstreifens (1) befindet, dicht bei einer vormarkierten Falte (13), nah am Bereich welcher dazu vorgesehen ist, eine Einheit anzuschließen, welche aus der Stütze (10) und dem Emittersensor (6) gebildet ist, welche visuelle In-situ-Warnungen mittels LEDs (9) erzeugt, wenn ein Steuerknopf (7) gedrückt wird und welche dazu programmiert ist, Daten auf ein Steuerzentralenpaneel mittels einer drahtlosen Verbindung kontinuierlich zu übertragen, Normalitätsinformation und Information bezüglich der Bedürfnis nach Aufmerksamkeit aufgrund einer nassen Windel (5.1) zu vermitteln, wobei der Emittersensor (6) innerlich die elektronische Schaltung zum Ablesen der Widerstände zwischen dem Leiter (2), dem Alarmgeber/-emitter und der Stromversorgungsbatterie aufnimmt, äußerlich aufweisend den Steuerknopf (7), eine, zwei oder mehr Indikator-LEDs (9) und zwei Anschlussklemmen (8), welche funktionieren, während sie in ein klammerartiges Stützelement (10) eingeführt sind, um dieselbe auf Windeln, Damenbinden, Unterlagen oder einem anderen Kleidungsstück zu immobilisieren.

2. Urindetektionsvorrichtung mit Klebeband und einem Emittersensor nach Anspruch 1, **dadurch gekennzeichnet, dass** der Bandstreifen (1) als gewickelte Bandrolle (1.1) vorliegt, mit einer Vielzahl von Widerständen (11), welche an die Leiter (2) angeschlossen sind, getrennt mit einem Abstand von etwa 50 Zentimetern mit vormarkierten Falten (13) und vormarkierten Schnitten (13.1).

3. Urindetektionsvorrichtung mit Klebeband und einem Emittersensor nach den Ansprüchen 1 und 2, **dadurch gekennzeichnet, dass** die Bandstreifen (1) oder die Bandrolle (1.1) aus Textilmaterial, Kunststoffmaterial, Vliesstoff oder einem anderen Material hergestellt sind, und die beiden Leiter (2), welche miteinander über eine deren Flächen durch irgendeine Methode integral verbunden sind, aus Kupfer, Aluminium, Stahl oder einem ähnlichen Metall hergestellt sind, wobei die gegenüberliegende Fläche der Bänder eine Klebeschicht (3) aufweist, wobei die genannte Schicht durchgängig oder diskret, aufgrund der Anwesenheit von Nuten oder Linien, um deren Durchlässigkeit zu erhöhen, ist.

4. Urindetektionsvorrichtung mit Klebeband und einem Emittersensor nach den vorhergehenden Ansprüchen, **dadurch gekennzeichnet, dass** die Leiter (2) des Bandstreifens (1) oder der Bandrolle (1.1) Kontakt mit dem Emittersensor (6) über die Anschlussklemmen (8) herstellt.

5. Urindetektionsvorrichtung mit Klebeband und einem Emittersensor nach den vorhergehenden Ansprüchen, **dadurch gekennzeichnet, dass** der Bandstreifen (1) in Segmenten vorliegt, welche Längen von etwa 50 Zentimetern aufweisen, wobei die Klebeschicht (3) mit einer Schutzfolie aus Papier oder Kunststoff geschützt ist.

## Revendications

1. Dispositif pour la détection d'urine avec ruban adhésif et capteur émetteur, le dispositif étant **caractérisé en ce qu'**il comprend une bande de ruban (1) équipée de deux conducteurs métalliques parallèles, longitudinaux, parallèles (2) auxquels est reliée une résistance de 90 KΩ (11) au moyen de superposition, adhérence ou impression, la résistance étant située à une extrémité de la bande de ruban (1), à proximité d'un pli pré-marqué (13), près d'une zone destinée à connecter une unité formée par le support (10) et le capteur émetteur (6), qui produit *in situ* des avertissements visuels au moyen de LEDs (9) lorsqu'un bouton de contrôle (7) est pressé et qui est programmé pour transmettre en continu des données à un panneau central de contrôle au moyen d'une connexion sans fil, pour faciliter des informations sur la normalité et des informations concernant le besoin d'attention à cause d'une couche mouillée (5.1), où le capteur émetteur (6) loge intérieurement le circuit électronique pour lire les résistances entre le conducteur (2), le générateur/émetteur d'alarme et la batterie d'alimentation présentant extérieurement le bouton de contrôle (7), une, deux ou plusieurs indicateurs LEDs (9) et deux bornes de connexion (8) fonctionnant pendant qu'elles sont insérées dans un élément de support de type pince (10) pour immobiliser celui-ci sur des couches, des serviettes hygiéniques, des compresses ou tout autre vêtement.

2. Dispositif pour la détection d'urine avec ruban adhésif et capteur émetteur selon la revendication 1, **caractérisé en ce que** la bande de ruban (1) se présente comme un rouleau de ruban enroulé (1.1), avec une pluralité de résistances (11) qui sont connectées aux conducteurs (2), séparés par une distance d'environ 50 centimètres avec des plis pré-marqués (13) et des coupes pré-marquées (13.1).

3. Dispositif pour la détection d'urine avec ruban adhésif et capteur émetteur selon les revendications 1 et 2, **caractérisé en ce que** les bandes de ruban (1) ou le rouleau de ruban (1.1) sont faits en matière textile, matière plastique, tissu non tissé ou toute autre matière, et les deux conducteurs (2), qui sont intégralement reliés ensemble par l'une de leurs faces par n'importe quelle méthode, sont faits en cuivre, aluminium, acier ou un métal similaire, dans lequel la face opposée des rubans a une couche adhésive (3), ladite couche étant continue ou discrète en raison de la présence d'encoches ou de lignes destinées à augmenter sa perméabilité.

4. Dispositif pour la détection d'urine avec ruban adhésif et capteur émetteur selon les revendications précédentes, **caractérisé par le fait que** les conducteurs (2) de la bande de ruban (1) ou du rouleau de ruban (1.1) établissent un contact avec le capteur émetteur (6) à travers des bornes de connexion (8).

5. Dispositif pour la détection d'urine avec ruban adhésif et capteur émetteur selon les revendications précédentes, **caractérisé en ce que** la bande de ruban (1) est présentée en segments ayant une longueur d'environ 50 centimètres, la couche adhésive (3) étant protégée par une feuille de protection en papier ou en plastique.
